# EUROPEAN PATENT SPECIFICATION

(11) **EP 1 608 347 B1**
(45) Date of publication and mention of the grant of the patent: **13.08.2014**
(21) Application number: 04724095.7
(22) Date of filing: 29.03.2004
(51) Int. Cl.: A61K 9/50, A61K 9/54

(54) **SOLID ORAL DOSAGE FORM CONTAINING SEAMLESS MICROCAPSULES**
SOLIDE ORALE DOSIERFORM MIT NAHTLOSEN MIKROKAPSELN
FORME POSOLOGIQUE SOLIDE S'ADMINISTRANT PAR VOIE ORALE QUI CONTIENT DES MICROCAPSULES SANS SOUDURE

(30) Priority: 28.03.2003 US 458599 P
(43) Date of publication of application: 28.12.2005
(60) Divisional application: 11165235.0
(73) Proprietor: Sigmoid Pharma Limited, Dublin 9 (IE)
(72) Inventor: MOODLEY, Joey, Athlone County Westmeath (IE); COULTER, Ivan, Dublin 2 (IE)
(74) Representative: Couchman, Jonathan Hugh
(86) International application number: PCT/IE2004/000049
(87) International publication number: WO 2004/084870

(56) References cited:
- EP-A- 0 778 083
- EP-A1- 0 573 978
- EP-A1- 1 184 038
- EP-A2- 0 525 731
- WO-A-00/69420
- US-A- 4 597 959
- US-A- 5 871 774
- US-A- 6 022 562

## Description

### Field Of The Invention

The present invention relates to a solid oral dosage form comprising a multiplicity of seamless microcapsules containing a pharmaceutically active ingredient solubilised in a pharmaceutically acceptable solvent or liquid phase.

### Background

Drugs for use in therapy and prophylaxis of various medical conditions have varying solubility characteristics ranging from insoluble to lipid soluble and water soluble with varying pH sensitivities. This variation in solubility can affect the therapeutic effectiveness of drugs. Drug dissolution is a prerequisite to drug absorption. Except in very special cases, drugs cannot be absorbed until they are solubilised. If a drug is already in solution at the time of administration, its absorption across the gastrointestinal tract and hence its bioavailability is rapid resulting in a fast therapeutic effect. Rapid or instantaneous bioavailability is characterised by significant blood levels within about 10 to 60 minutes after administration of the drug.

Drugs that are poorly water soluble and/or sensitive to pH must be formulated in a way that improves their solubility and hence their bioavailability. In general, a drug that is in solution or suspension when administered by the oral route is rapidly, and frequently instantaneously, absorbed from the gastrointestinal tract resulting in a fast therapeutic action. However, in many cases, it is desirable to control the rate of drug absorption following oral administration in order to achieve the desired plasma profile or prolongation of action of the drug.

Numerous processes and formulations exist to enhance the solubility of poorly soluble drug compounds. Solubilisation of such drugs in solvents, oils, emulsions and microemulsions are well known to those skilled in the art and have been used to deliver such drugs orally. Such formulations are then encapsulated in soft gelatine capsules for oral administration. Soft gelatine encapsulation is a specialised process, whereby the end products, soft gelatine capsules do not lend themselves easily to further processing such as the addition of delayed or sustained release coatings. Therefore such dosage forms are particularly suited for instantaneous delivery of the encapsulated active drugs.

Particle size reduction processes such as milling, micronisation and spray drying are also well known to increase the surface area and hence solubilisation rates of poorly soluble drugs. U.S. Pat. No. 5,510,118 and US Pat. No. 5,145,684 teaches the milling of such drugs in pharmaceutically acceptable solvents to produce nanosuspensions of the drugs with the resulting enhanced solubility of the drug. The production of nanosuspension/nanodispersion of poorly soluble drugs can be performed by using a double nozzle spray drying process. These nanosuspensions or nanodispersions result in increased dissolution rates of the active and increased absorption and bioavailability. However for administration as a solid oral dosage form with controlled and/or sustained release characteristics, the nanodispersions or nanosuspensions are subsequently dried to produce free flowing powder for further processing into a solid oral dosage form such as tablets, minitablets which can then be coated with a controlled or sustained release polymers to control the delivery rate of the active. Recovery of the nanoparticle size distribution on dissolution of these dosage forms is however limited often resulting in reduced dissolution rate of the active.

There is a need therefore for an oral formulation or process which can be used to administer the solubilised and/or dispersions including nanosuspensions of the active ingredients in a manner which allows the formulation to be subsequently coated to deliver the active ingredient at a predetermined site of absorption and /or at a predetermined rate of delivery consistent with the optimum absorption and bioavailabilty or plasma profile of the drug.

Additionally there is a need for an oral formulation which can be used to administer one or more active ingredients of differing solubility which are released in a predetermined manner to target sites in the gastrointestinal tract so as to achieve maximum absorption at the site of release of active ingredient.

There is also a need for an oral formulation which allows for release of an active ingredient in the gastrointestinal tract in a manner which minimises high local concentrations of solid active ingredient. Multiparticulate drug delivery systems by their nature allow the release of the active ingredient over a larger surface area of the gastrointestinal tract thereby minimising high localised drug concentration for drugs which are irritants to the gastrointestinal tract.

Drugs which are poorly permeable are often administered with one or more permeability enhancers to enhance their permeability and absorption. Numerous potential absorption enhancers have been identified. Medium chain fatty acids and triglycerides have demonstrated the ability to enhance the absorption of hydrophilic drugs across the gastrointestinal mucosa (Pharm.Res., 1994,11,1148-54), (Pharm. Res.1993, 10,857-864). U.S. Patent No. 4,656,161 (BASF AG) discloses a process for increasing the bioavailability of heparins and heparinoids by adding non-ionic surfactants with a fatty acid, a fatty alcohol, an alkylphenol or a sorbitan or glycerol fatty acid ester. It is also known that the administration of medium chain fatty acids and derivatives of, including amino acid derivatives in combination with the drug can enhance permeability of the drug.

For maximum enhancement, it is desirable that both enhancer and drug are in solution at the same rate. The solubilities of the enhancer and drug are often different resulting in different rates of solubilisation of the enhancer and drug and hence loss of bioavailability of the drug in comparison with bioavailability from a solution of the drug and enhancer. In addition poorly permeable drugs including macromolecular drugs and biotechnology drugs such as peptides, proteins, vaccines, oligosaccharides, polysaccharides including TRH, Calcitonin, Leoprolide acetate, alendronate, vasopressin, desmopressin and antisense oligonucleotides are acid labile and cannot be delivered to the stomach as a solution.

The use of essential oils to enhance bioavailability by reducing cytochrome P450 metabolism and/or P-glycoprotein regulated transport of the active out of the blood stream back into the gut is also known, (US 5,66,386 to AvMax Inc. and others). EP 1184038 discloses a system whereby a substance which is orally taken is selectively delivered to the lower digestive tract. Compositions disintegrating in the lower digestive tract are characterized by containing a compound <A>, which has a molecular weight of 1000 or less and has a disulfide bond, and a polymer, which has a molecular weight exceeding 1000 and is digested by enteric bacteria and/or undergoes softening, swelling or dissolution due to a decrease in pH.

There is a need therefore for a controlled release technology which will allow the delivery of a drug and enhancer in solution to the optimum site of absorption/action in the gastrointestinal tract.

### Summary Of The Invention

The invention provides a controlled release formulation in solid unit dosage form, said formulation comprising a multiplicity of seamless microcapsules, each of which microcapsule comprises:
a shell having a polymeric composition formed of a soft or hard gelatine; and
a core which contacts the shell and is a solution of one or more active ingredients dissolved in a glyceride selected from medium chain mono-, diand/or tri- glycerides which comprises a surfactant as an auxiliary agent
and each microcapsule having a predetermined release rate of active ingredient in the gastrointestinal tract following administration, the microcapsules collectively have one or more rates of release of active ingredient dependent on a predetermined permeability of the respective microcapsules.

The microcapsules can be administered as a sprinkle on food or filled into a hard gelatine capsule or tabletted as a matrix, bilayer or multiple layer tablets. The final dosage form can be further coated to release the microcapsules at predetermined rates and/or site of the gastrointestinal tract.

The microcapsules of the formulation according to the invention effectively allow one to administer solutions of active ingredients as if they were multiparticulate solid oral dosage forms. The microcapsules release their contents to the gastrointestinal tract in a manner which minimises high local concentrations of active ingredient which might otherwise result in irritation and other undesirable side effects, but additionally the drug is released in an already solubilised form which aids absorption.

The individual microcapsules suitably have an average diameter in the range 100-10,000µm, more particularly in the range 250-8,000µm and especially in the range 500-5,000µm.

The walls of the microcapsules will suitably have a thickness in the range 30-1,000µm, especially in 100-500µm.

The walls of the microcapsules have a predetermined permeability by which is meant they either dissolve or have a natural permeability to gastrointestinal fluid so that active ingredient is released therefrom as desired in the gastrointestinal tract.

Microcapsules with naturally permeable walls can be soluble, porous or, alternatively, the solubility or porosity can develop as a result of the change in environmental conditions as the formulation passes through the gastrointestinal tract.

The wall of the microcapsule is formed of soft or hard gelatine (such as a bovine or porcine gelatine material) or other soft gel materials made from suitable polymers. Examples of other soft gel materials include starches that form a soft gel or high molecular weight polyethylene glycols or Agar/Agar. However, in practice any material that can dissolve in the gastrointestinal fluid can be used. Such a material can be a material which is incorporated in the wall and which dissolves in the gastrointestinal fluid, namely a porosigen.

The wall of the microcapsule will have a polymeric composition and/or structure which allows for fast release and thus fast absorption of active ingredient once the wall is partially or wholly permeable.

Once the wall of the microcapsule has the requisite permeability, the active ingredient in its liquid carrier passes into the gastrointestinal tract for immediate absorption. The liquid carrier can include one or more auxiliary agents with bioavailability and/or cytoprotective, especially gastroprotective, enhancing properties.

The formulation according to the invention can comprise a blend of microcapsules having walls of variable, but predetermined permeability so as to achieve immediate, intermediate or sustained release of active ingredient over a given time period in the gastrointestinal tract.

Accordingly, it will be appreciated that the formulation according to the invention has advantages over conventional solid microparticles in which the release of active ingredient is dependent on progressive solubilisation in the gastrointestinal tract.

The permeability of the walls of the microcapsules can be dependent on pH, temperature and other physical conditions prevailing within the gastrointestinal tract.

The controlled release properties of the microcapsules according to the invention will principally be a function of the thickness of the walls of the microcapsules, or by including pH dependent substances such as polymers or shellac.

The microcapsules can contain two or more active ingredients having different solubilities in the aqueous environment of the gastrointestinal tract, but with compatible solubility capability in the liquid medium of the microcapsule. Alternatively, the microcapsules can contain single active ingredients solubilised in different media but which can be released for simultaneous absorption from microcapsules having walls of different materials but with similar or different permeability characteristics.

The microcapsules can also contain two or more active ingredients having different half lives following absorption from the gastrointestinal tract.

The solubility of the or each active ingredient can be dependent on the pH of a given region of the gastrointestinal tract.

The microcapsules can be manufactured so that they release their contents in the gastrointestinal tract at a point at which the drug is most soluble. This feature enables one to maximise absorption because the microcapsules release their contents when the pH conditions are optimal.

The pH internally of the microcapsules can be optimised by the use of an acid or an alkaline solution, as required to maximise the absorption of active ingredients that are pH sensitive.

An example of a formulation with microcapsules capable of achieving immediate, intermediate and sustained release of active ingredient over a given time period in the gastrointestinal tract might be a formulation for use in the treatment of the common cold and influenza. Such formulations are conventionally multiple active ingredient formulations. The common cold and influenza are each characterised by a variety of symptoms which cannot generally be alleviated by a single active ingredient. For example, it might be desired to administer an antihistamine, a decongestant and one or more cough suppressants.

It may also be desirable to add a diuretic such as hydrochlorothiazide and/or an anti hypertensive such as Losartan.

The formulation according to the present invention is ideally suited for use as a multiple active ingredient formulation.

The microcapsules can contain an active ingredient solubilised in a solution of a permeability enhancer in a ratio of enhancer to drug ranging from 0 drug: 100 enhancer to 100 drug : 0 enhancer which can be released for simultaneous absorption from microcapsules having walls of different materials but with similar or different permeability characteristics.

Additionally microcapsules containing the enhancer solution without the drug can be released at a slower rate from microcapsules containing the drug and enhancer solutions to maximise the permeability enhancement of the unabsorbed drug in the gastrointestinal tract.

The walls of the microcapsules can have inherent mucoadhesive properties and thus bind to the wall of the gastrointestinal tract during release of active ingredient therefrom.

The walls of the microcapsules can also have inherent enteric coating properties.

Some or all of the microcapsules in the formulation can have an enteric coating, for example, an outer membrane of shellac or other enteric coating.

It will be appreciated that one can achieve selective absorption of active ingredients using a formulation in accordance with the invention.

Essentially the solution in which the drug is dissolved is any pharmaceutically acceptable solvent or liquid phase provided the solvent or liquid phase does not dissolve the wall of microcapsules.

Such phase is short or medium chain mono-, di and/or tri- glycerides.

The core can also contain one or more auxiliary agents selected from a pH controlling agent, an anti-oxidant, a humectant, a surfactant and a vasodilator.

Suitable pH controlling agents include, for example, citric acid, fumaric acid, sodium citrate and the like.

Examples of anti-oxidants are sodium metabisulphite, butylated hydroxyanisole and butylated hydroxytoluene or a mixture thereof.

Examples of suitable humectants are glycerol and sorbitol.

Examples of suitable surfactants include sodium lauryl sulphate, diethylene glycol monostearate, propylene glycol monosteareate, polyethylene glycol, polysorbates and polyvinyl alcohol or mixtures thereof.

The microcapsules can contain up to 90% by weight of active ingredient. However, in general the microcapsules will contain between 25 and 75% by weight of active ingredient.

Each formulation will suitably contain between 10 and 300 microcapsules, preferably between 100 and 250 microcapsules.

The microcapsules will suitably be administered by loading them into a hard gelatine capsule which will be swallowed in the normal way or by loading them into another container such as a sachet, the contents of which can then be swallowed or sprinkled onto food and swallowed. In certain special circumstances the microcapsules may be incorporated into a tablet in a way which maintains their integrity.

A formulation in accordance with the invention can be designed so as to achieve fast, medium and slow release of one or more active ingredients. Thus, it will be appreciated that the formulation in accordance with the invention can be used to achieve maximum bioavailability resulting from maximum absorption of one or more active ingredients from the gastrointestinal tract.

The formulation according to the invention is suitable for the administration of a wide range of active ingredients.

For example the formulation can be used in the case of insoluble active ingredients such as nifedipine, lipid soluble active ingredients such as gemfibrizol, and pH sensitive active ingredients such as captopril.

The formulation according to the invention is also suitable for the administration of active ingredients which are sensitive to the pH environment in the stomach, such as omeprazole and other proton pump inhibitors used in anti -ulcer treatment

The formulation according to the invention can also be used to improve the bioavailability of active ingredients such as terfenadine which have a low oral bioavailability.

The formulation according to the invention can also be used to dramatically increase the absorption of active ingredients which are poorly absorbed from or are destroyed in the gastrointestinal tract such as captopril, cyclosporin, calcitonin, heparins and heparinoids.

Suitable classes of therapeutic agents which can be delivered using this invention include but are not limited to poorly water soluble drugs such as cardiovascular agents, lipid lowering agents, anti-diabetic agents, anti-epileptics, anti-infectives, anti-fungal agents, anti-viral agents, antipsychotic agents, immunosuppressants, protease inhibitors, cyclic peptides.

Suitable classes of therapeutic agents which can be delivered using this invention include but are not limited to peptides, proteins, vaccines, and oligonucleotides, including non-covalent or covalent modified versions thereof.

An example of a formulation of an insoluble drug providing both immediate and sustained release in accordance with the invention comprises a capsule containing a mixture of immediate and sustained release seamless microcapsules of varying sizes, for example. 1-8mm. The active ingredient, for example, nifedipine or cyclosporine, is dissolved, for example in a suitable combination of an oil and surfactants. An outer membrane liquid for example, gelatin and an inner active ingredient liquid are combined to form droplets i.e., microcapsules. The microcapsules are passed through a cooling system, for example, oil. The seamless microcapsules containing the active ingredient are removed from the cooling system and cleaned and dried in separate facilities.

The sustained action of the microcapsules is determined by the thickness of the outer membrane or by the addition of a variety of sustained release polymers to the inner liquid or the outer membrane liquid, for example, polymethacrylates, cellulose derivatives, polyvinylpyrrolidones. As indicated above, the microcapsules can be formulated to release the active liquid ingredient at a specific absorption site, for example by the use of pH dependent polymethacrylates.

The invention provides an oral formulation which allows for protection of the active ingredient from harsh environments such as gastric acid and intestinal proteases and other degradative processes. Enteric coating protects drugs from release into acidic environments, protease and nuclease inhibitors reduce proteolytic and nucleic acid degradation while mucoadhesive coatings minimise exposure to degradative enzymes.

For maximum protection from degradative processes, it may be desirable that the drug to be protected is formulated as a solution or emulsion that contains the appropriate degradation inhibitor, such as a nuclease or protease inhibitor. Otherwise, it may be necessary to formulate the drug and degradative process inhibitor as separate minicapsules. In addition, it may be necessary to coat the minicapsules with both an outer enteric coat and an inner mucoadhesive coating to reduce the exposure time to degradative enzymes thereby mitigating degradation.

### Brief Description of the Drawing

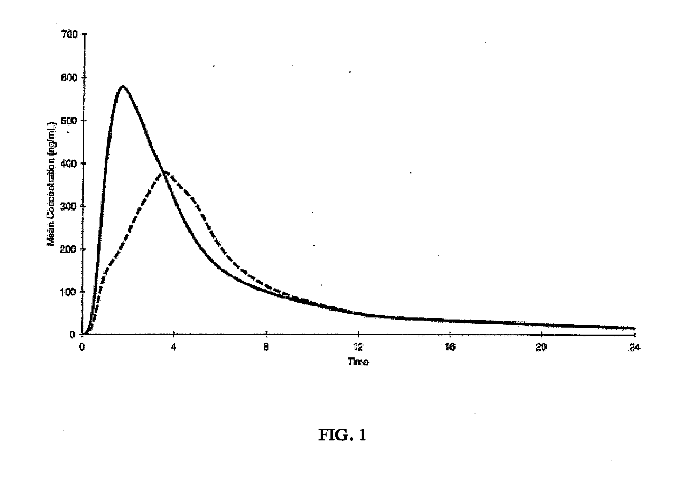

The invention will be more clearly understood from the following descriptions thereof, given by way of example only, with reference to the accompanying drawing, in which: -
Fig. 1 is a graph showing the bioavailability of the product over time.

The invention will be further illustrated by the following examples:

### General Outline of Seamless Microcapsule Formation

The principle of seamless microcapsule formation is the utilisation of "surface tension," when two different solutions (which are not or hardly dissolved with each other) contact each other, which works by reducing the contact area of the two different solutions.

After encapsulating the core solution, which is ejected through an orifice with a certain diameter, with the shell solution which is also ejected through an outer orifice, the encapsulated sphere is then ejected into a cooling or hardening solution and the outer shell solution is gelled or solidified. Thus seamless microcapsules are formed.

The core solution is mainly a hydrophobic solution. The outer shell solution is normally gelatin based.

With the nozzle having a single orifice, a capsule or a bead of shell/core mixed suspension can be processed. With the nozzle having two orifices (center and outer), a hydrophobic solution can be encapsulated. With the nozzle having three or more orifices seamless microcapsules for various applications can be processed.

### Reference Example 1

Nifedipine is dissolved in soybean oil and formed into seamless microcapsules according to the methods described in US Pat. Nos. 5,478,508 and 5,882,680 with an outer gelatin coating as hereinbefore described. These microcapsules have a size distribution of 1-3mm.

The nifedipine microcapsules are then coated in a conventional manner with a cellulose polymer coating to provide a controlled release dissolution rate.

The coated microcapsules are finally encapsulated into a hard gelatin capsule shell.

The details for this example are as follows:

| *Core Solution* | *Weight %* |
|---|---|
| -- Nifedipine USP | 9.1 |
| -- Polyethylene Glycol (Grade 200;300;400;600) | 90.9 |

| *Intermediate Solution* | |
|---|---|
| -- Vegetable Oil | 100.0 |

| *Film Solution* | |
|---|---|
| --Gelatin | 12.0 - 22.5 |
| -- Sorbitol | 1.5 - 2.5 |
| -- Purified Water | 75 - 85 |

| *Microcapsule Formation* | |
|---|---|
| *Ingredients* | *Weight %* |
| -- Core Solution | 30-70 |
| -- Intermediate | Solution 5-25 |
| -- Film Solution | 15-65 |

The core solution is formed into seamless microcapsules with an intermediate solution layer and an outer hard gelatin film layer. The intermediate layer acts as a barrier preventing the core solution from migrating into the outer gelatin layer.

The microcapsules produced have a particle size range of between 1.00 - 3.00 mm.

### Example 1

Gemfibrozil (a liquid soluble drug) is formulated along with various surfactants and gelatin into seamless microcapsules of varying thickness.

A portion of these pellets are coated with a methacrylate polymer system and combined in a ratio of 4:1 with uncoated microcapsules, then filled into hard gelatin capsule shells, thereby providing a drug formulation having both immediate and sustained release dissolution characteristics.

### Reference Example 2

Nimodipine is solubilised in a suitable solvent such as PEG 400 and formed into seamless microcapsules with an outer gelatin coating as hereinbefore described. These microcapsules have a size distribution of 3-6mm.

The nimodipine microcapsules are then encapsulated into a hard gelatin capsule shell.

### Reference Example 3

Nimodipine seamless microcapsules are formulated as described in Reference Example 2, however, the inherent release characteristics of the pellets are varied from Reference Example 2, by increasing the wall thickness of the microcapsules.

These microcapsules, when coated with the same polymer coating as in Reference Example 1, provide a longer controlled release action.

### Example 2

Cyclosporine is solubilised in a suitable medium chain triglyceride to which is added bile salt enhancer or bile salts, and formed into seamless microcapsules with an outer gelatin coating as herein before described. These microcapsules have a size distribution of 3-6mm.

The cyclosporine and bile salt enhancer or bile salts microcapsules are then encapsulated into a hard gelatin capsule shell to give a cyclosporine content of 25-50mg /capsule.

### Example 3

| | |
|---|---|
| Cyclosporine USP | 100 |
| Labrifil M1944 | 50-300 |
| Cremophor RH40 | 50-400 |
| Tween 80 | 0-200 |
| Polyethylene Glycol (grade 200;300;400;600) | 50-200 |
| Ethanol | --- |

Place labrifil, cremophor and tween (if required) in a suitable container and mix using a mechanical mixer until uniformly dispersed or dissolved.(Label as Mix 1)

Place polyethylene glycol and ethanol (as required) in a suitable container. Add cyclosporine and mix using a mechanical mixer until the cyclosporine is completely solubilised.(Label as Mix 2)

Add Mix 1 to Mix 2 and continue mixing until all ingredients are uniformly dispersed. This is the Active Core Solution.

The active core solution is processed in the same way as described in reference example to form seamless microcapsules having a particle size range of 1.50-2.00mm.

The cyclosporine microcapsules are then encapsulated into hard gelatin capsules.

### Example 4

The microcapsules of Example 3 were administered to 8 healthy male volunteers and the bioavailabilty was compared with a conventional soft gel formulation of cyclosporin which is available under the Trade Mark Sandimmune from Novartis. The resulting bioavailabity data demonstrate that the Tmax was reduced from 3.25 hr to 1.75 hr (100% increased speed to uptake) and the Cmax was increased from 580ng/ml to 750ng/ml (>40% increased drug uptake) when compared with the conventional formulation. Overall the AUC (area under the curve) was increased more than 20% when compared with the reference formulation. The data is presented in the following table. The bioavailability data are illustrated in Fig. 1 in which the continuous line represents bioavailability of the product of Example 3 and the interrupted line is bioavailability of the conventional reference product.

| PK Parameters | | **Formulation of example 19** | **Reference** | **Summary** |
|---|---|---|---|---|
| **Relative Bioavailability** | **Based on** | **103.14 ±44.62** | | **-** |
| **(%)** | **AUCinf** | **43.3** | | **-** |
| **AUCinf (ng/mL.h)** | | **3040.00 ± 1091.71** | **2586.23 ± 341.15** | **> 20% Increase** |
| **CV%** | | **35.9** | **13.2** | |
| **AUClast (ng/mL.h)** | | **2855.09 ± 1043.08** | **2410.68 ± 332.93** | **> 20% Increase** |
| **CV%** | | **36.5** | **13.8** | |
| **Cmax (ng/mL)** | | **732.31 ± 273.67** | **581.06 ± 109.13** | **∼40% Increase** |
| **CV%** | | **37.4** | **18.8** | |
| **Tmax(h)** | | **2.00 ± 0.93** | **3.00 ± 1.31** | **100%** |
| **CV%** | | **46.3** | **43.6** | **Faster** |
| **Median Range** | | **1.75 (1.00-3.50)** | **3.25 (1.00-5.00)** | |
| **Thalf** | | **8.12 ± 0.50** | **7.95 ± 1.30** | **Equivalent** |
| **(h)** | | | | |
| **CV%** | | **6.1** | **16.3** | |

The present invention allows for an active pharmaceutical ingredient to be maintained in a liquid phase and then encapsulated into microcapsules. The present invention also allows the active ingredient to be maintained in its liquid phase thus enhancing the solubility of an insoluble partially soluble pharmaceutical ingredient.

## Claims

1. A controlled release formulation in solid unit dosage form, said formulation comprising a multiplicity of seamless microcapsules, each microcapsule comprising:
a shell having a polymeric composition formed of a soft or hard gelatine ; and
a core which contacts the shell and is a solution of one or more active ingredients dissolved in a glyceride selected from medium chain mono-, di- and/or tri- glycerides which comprises a surfactant as an auxiliary agent
and each microcapsule having a predetermined release rate of each said active ingredient in the gastrointestinal tract following administration, the microcapsules collectively having one or more rates of release of each said active ingredient dependent on a predetermined permeability of the respective microcapsules.

2. A formulation of claim 1 wherein the shells of the microcapsules additionally include a sustained release polymer.

3. A formulation of claim 1 or claim 2 wherein some or all of the microcapsules in the formulation have an enteric coating.

4. A formulation of any preceding claim wherein the microcapsules have an average diameter in the range of 0.5-5mm.

5. A formulation of any preceding claim wherein the surfactant is sodium lauryl sulphate, diethylene glycol monostearate, propylene glycol monosteareate, polyethylene glycol, polysorbates and polyvinyl alcohol or a mixture thereof.

6. A formulation of claim 1 wherein the microcapsules further comprise a cellulose polymer coating.

7. A formulation of any preceding claim wherein the active ingredient is a pharmaceutical ingredient.

8. A formulation of claim 7 wherein the active ingredient is selected from nifedipine, gemfibrizol, terfenadine, captopril, cyclosporin, calcitonin, heparins, heparinoids, amlodipine and nimodipine.

9. A formulation of claim 8 wherein the active ingredient is cyclosporin.

10. A formulation of claim 8 wherein the active ingredient is nimodipine.

11. A formulation of claim 7 wherein the active ingredient is a therapeutic agent selected from peptides, proteins, vaccines, and oligonucleotides.

12. A formulation of any of claims 1 to 7 which is a multiple active ingredient formulation.

13. A formulation of any of claims 1 to 7 wherein the microcapsules contain two or more active ingredients having different solubilities in the aqueous environment of the gastrointestinal tract, but with compatible solubility in the liquid medium of the microcapsule.

14. A formulation of any of claims 1 to 7 wherein the microcapsules contain two or more active ingredients having different half lives following absorption from the gastrointestinal tract.

15. A seamless microcapsule comprising:
a shell having a polymeric composition formed of a soft or hard gelatine; and
a core which contacts the shell and is a solution of one or more active ingredients dissolved in a glyceride selected from medium chain mono-, di-and/or tri-glycerides which comprises a surfactant as an auxiliary agent
and each microcapsule having a predetermined release rate of each said active ingredient in the gastrointestinal tract following administration, the microcapsules collectively having one or more rates of release of each said active ingredient dependent on a predetermined permeability of the respective microcapsules.

16. A microcapsule of claim 15 wherein the shell additionally includes a sustained release polymer.

17. A microcapsule of claim 15 or claim 16 which has an enteric coating.

18. A microcapsule of any of claims 15 to 17 which has a diameter in the range of 0.5-5mm.

19. A microcapsule of any of claims 15 to 18 wherein the surfactant is sodium lauryl sulphate, diethylene glycol monostearate, propylene glycol monosteareate, polyethylene glycol, polysorbates and polyvinyl alcohol or a mixture thereof.

20. A microcapsule of claim 15 which further comprises a cellulose polymer coating.

21. A microcapsule of any of claims 15 to 20 wherein the active ingredient is a pharmaceutical active ingredient.

22. A microcapsule of claim 21 wherein the active ingredient is selected from nifedipine, gemfibrizol, terfenadine, captopril, cyclosporin, calcitonin, heparins, heparinoids, amlodipine and nimodipine.

23. A microcapsule of claim 22 wherein the active ingredient is cyclosporin.

24. A microcapsule of claim 22 wherein the active ingredient is nimodipine.

25. A microcapsule of claim 21 wherein the active ingredient is a therapeutic agent selected from peptides, proteins, vaccines, and oligonucleotides.

26. A method of forming a seamless microcapsule of claim 15, comprising:
combining to form droplets (i) a hydrophobic core solution of the one or more active ingredients dissolved in a glyceride selected from mono-, di-, or tri-glycerides which comprises a surfactant as an auxiliary agent and (ii) a gelatine-based shell solution; and
passing the droplets through an oil or other cooling system.

27. A method of claim 26, comprising ejecting through an orifice a hydrophobic core solution,of one or more active ingredients dissolved in a pharmaceutically acceptable liquid phase which comprises a surfactant as an auxiliary agent, and ejecting through an outer orifice a gelatine-based shell to form an encapsulated sphere, the sphere then being ejected into a cooling or hardening solution and the outer shell solution being gelled or solidified.

28. A method of claim 26, comprising ejecting through a nozzle having a single orifice a shell/core mixed suspension, the core being a hydrophobic core solution of one or more active ingredients dissolved in a pharmaceutically acceptable liquid phase which comprises a surfactant as an auxiliary agent, the shell being a gelatine-based shell solution, to form a capsule or a bead of shell/core mixed suspension which is then ejected into a cooling or hardening solution and the shell solution is gelled or solidified.

29. A method of any of claims 26 to 28 wherein the shell solution additionally includes a sustained release polymer.

30. A method of any of claims 26 to 29 which further comprises coating the microcapsule with an enteric coating.

31. A method of any of claims 26 to 28 wherein the microcapsule has a diameter in the range of 0.5-5mm.

32. A method of any of claims 26 to 31 wherein the surfactant is sodium lauryl sulphate, diethylene glycol monostearate, propylene glycol monosteareate, polyethylene glycol, polysorbates and polyvinyl alcohol or a mixture thereof.

33. A method of claim 26 which further comprises coating the microcapsule with a cellulose polymer coating.

34. A method of any of claims 26 to 33 wherein the active ingredient is a pharmaceutical active ingredient.

35. A method of claim 34 wherein the active ingredient is selected from nifedipine, gemfibrizol, terfenadine, captropril, cyclosporin, calcitonin, heparins, heparinoids, amlodipine and nimodipine.

36. A method of claim 35 wherein the active ingredient is cyclosporin.

37. A formulation of claim 35 wherein the active ingredient is nimodipine.

38. A method of claim 34 wherein the active ingredient is a therapeutic agent selected from peptides, proteins, vaccines, and oligonucleotides..

39. A method of any of claims 26 to 38 which further comprises formulating the microcapsule into a solid unit dosage form comprising a multiplicity of such seamless microcapsules.

## Patentansprüche

1. Retardformulierung in fester Darreichungsform, wobei die Formulierung eine Mehrzahl von nahtlosen Mikrokapseln umfasst, wobei jede Mikrokapsel folgendes umfasst:
eine Schale mit einer polymeren Zusammensetzung, gebildet aus weicher oder harter Gelatine; und
einen Kern, der die Schale berührt und eine Lösung eines oder mehrerer Wirkstoffe ist, aufgelöst in einem Glycerid ausgewählt aus mittelkettigen Mono-, Diund/oder Triglyceriden, ein Tensid als Hilfsstoff umfassend;
und wobei jede Mikrokapsel eine vorbestimmte Freisetzungsrate für jeden Wirkstoff im Magen-Darm-Trakt nach der Verabreichung aufweist, wobei die Mikrokapseln gemeinsam eine oder mehrere Freisetzungsraten jedes Wirkstoffs aufweisen, abhängig von einer vorbestimmten Permeabilität der entsprechenden Mikrokapseln.

2. Formulierung nach Anspruch 1, wobei die Schalen der Mikrokapseln ein Polymer mit retardierter Freisetzung aufweisen.

3. Formulierung nach Anspruch 1 oder 2, wobei einige oder alle Mikrokapseln in der Formulierung einen enterischen Überzug aufweisen.

4. Formulierung nach einem der vorstehenden Ansprüche, wobei die Mikrokapseln einen durchschnittlichen Durchmesser im Bereich von 0,5 bis 5 mm aufweisen.

5. Formulierung nach einem der vorstehenden Ansprüche, wobei das Tensid Natriumlaurylsulfat, Diethylenglycolmonostearat, Propylenglycolmonostearat, Polyethylenglycol, Polysorbate und Polyvinylalkohol oder eine Mischung dieser ist.

6. Formulierung nach Anspruch 1, wobei die Mikrokapseln ferner einen Zellulosepolymerüberzug umfassen.

7. Formulierung nach einem der vorstehenden Ansprüche, wobei der Wirkstoff ein pharmazeutischer Wirkstoff ist.

8. Formulierung nach Anspruch 7, wobei der Wirkstoff ausgewählt wird aus Nifedipin, Gemfibrizol, Terfenadin, Captopril, Cyclosporin, Calcitonin, Heparinen, Heparinoiden, Amlodipin und Nimodipin.

9. Formulierung nach Anspruch 8, wobei der Wirkstoff Cyclosporin ist.

10. Formulierung nach Anspruch 8, wobei der Wirkstoff Nimodipin ist.

11. Formulierung nach Anspruch 7, wobei der Wirkstoff ein therapeutischer Wirkstoff ist, ausgewählt aus Peptiden, Proteinen, Vakzinen und Oligonukleotiden.

12. Formulierung nach einem der Ansprüche 1 bis 7, wobei es sich um eine Formulierung mit mehreren Wirkstoffen handelt.

13. Formulierung nach einem der Ansprüche 1 bis 7, wobei die Mikrokapseln zwei oder mehr Wirkstoffe mit unterschiedlichen Löslichkeiten in der wässerigen Umgebung des Magen-Darm-Trakts aufweisen, jedoch eine kompatible Löslichkeit in dem flüssigen Medium der Mikrokapsel.

14. Formulierung nach einem der Ansprüche 1 bis 7, wobei die Mikrokapseln zwei oder mehr Wirkstoffe mit unterschiedlichen Halbwertzeiten nach Absorption durch den Magen-Darm-Trakt aufweisen.

15. Nahtlose Mikrokapsel, umfassend:
eine Schale mit einer polymeren Zusammensetzung, gebildet aus weicher oder harter Gelatine; und
einen Kern, der die Schale berührt und eine Lösung eines oder mehrerer Wirkstoffe ist, aufgelöst in einem Glycerid ausgewählt aus mittelkettigen Mono-, Diund/oder Triglyceriden, ein Tensid als Hilfsstoff umfassend;
und wobei jede Mikrokapsel eine vorbestimmte Freisetzungsrate für jeden Wirkstoff im Magen-Darm-Trakt nach der Verabreichung aufweist, wobei die Mikrokapseln gemeinsam eine oder mehrere Freisetzungsraten jedes Wirkstoffs aufweisen, abhängig von einer vorbestimmten Permeabilität der entsprechenden Mikrokapseln.

16. Mikrokapsel nach Anspruch 15, wobei die Schale zusätzlich ein Polymer mit retardierter Freisetzung aufweist.

17. Mikrokapsel nach Anspruch 15 oder 16, welche einen enterischen Überzug aufweist.

18. Mikrokapsel nach einem der Ansprüche 15 bis 17, welche einen Durchmesser im Bereich von 0,5 bis 5 mm aufweist.

19. Mikrokapsel nach einem der Ansprüche 15 bis 18, wobei das Tensid Natriumlaurylsulfat, Diethylenglycolmonostearat, Propylenglycolmonostearat, Polyethylenglycol, Polysorbate und Polyvinylalkohol oder eine Mischung dieser ist.

20. Mikrokapsel nach Anspruch 15, wobei diese ferner einen Zellulosepolymerüberzug umfasst.

21. Mikrokapsel nach einem der Ansprüche 15 bis 20, wobei der Wirkstoff ein pharmazeutischer Wirkstoff ist.

22. Mikrokapsel nach Anspruch 21, wobei der Wirkstoff ausgewählt wird aus Nifedipin, Gemfibrizol, Terfenadin, Captopril, Cyclosporin, Calcitonin, Heparinen, Heparinoiden, Amlodipin und Nimodipin.

23. Mikrokapsel nach Anspruch 22, wobei der Wirkstoff Cyclosporin ist.

24. Mikrokapsel nach Anspruch 22, wobei der Wirkstoff Nimodipin ist.

25. Mikrokapsel nach Anspruch 21, wobei der Wirkstoff ein therapeutischer Wirkstoff ist, ausgewählt aus Peptiden, Proteinen, Vakzinen und Oligonukleotiden.

26. Verfahren zum Bilden einer nahtlosen Mikrokapsel nach Anspruch 15, umfassend:
Kombinieren zur Bildung von Tröpfchen: (i) einer hydrophoben Kernlösung des einen oder der mehreren Wirkstoffe, aufgelöst in einem Glycerid ausgewählt aus mittelkettigen Mono-, Di- und/oder Triglyceriden, ein Tensid als Hilfsstoff umfassend und (ii) einer Schalenlösung auf Gelatine-Basis; und
Führen der Tröpfchen durch ein Öl- oder ein anderes Kühlsystem.

27. Verfahren nach Anspruch 26, umfassend das Ausstoßen durch eine Öffnung einer hydrophoben Kernlösung aus einem oder mehreren Wirkstoffen, aufgelöst in einer pharmazeutisch verträglichen flüssigen Phase, die ein Tensid als einen Hilfsstoff umfasst, und das Ausstoßen durch eine äußere Öffnung einer Schale auf Gelatinebasis, so dass eine gekapselte Sphäre gebildet wird, wobei die Sphäre danach in eine Kühl- oder Härtungslösung ausgestoßen wird, und wobei die äußere Schalenlösung geliert oder verfestigt wird.

28. Verfahren nach Anspruch 26, umfassend das Ausstoßen durch eine Düse mit einer einzigen Öffnung einer gemischten Suspension aus Schale und Kern, wobei der Kern eine hydrophobe Kernlösung aus einem oder mehreren Wirkstoffen ist, aufgelöst in einer pharmazeutisch verträglichen flüssigen Phase, die ein Tensid als einen Hilfsstoff umfasst, wobei die Schale eine Schalenlösung auf Gelatine-Basis ist, so dass eine Kapsel oder ein Kugel der gemischten Suspension aus Schale und Kern gebildet wird, die danach in eine Kühl- oder Härtungslösung ausgestoßen wird, und wobei die Schalenlösung geliert oder verfestigt wird.

29. Verfahren nach einem der Ansprüche 26 bis 28, wobei die Schalenlösung zusätzlich ein Polymer mit retardierter Freisetzung aufweist.

30. Verfahren nach einem der Ansprüche 26 bis 29, ferner umfassend das Überziehen der Mikrokapsel mit einem enterischen Überzug.

31. Verfahren nach einem der Ansprüche 26 bis 28, wobei die Mikrokapsel einen Durchmesser im Bereich von 0,5 bis 5 mm aufweist.

32. Verfahren nach einem der Ansprüche 26 bis 31, wobei das Tensid Natriumlaurylsulfat, Diethylenglycolmonostearat, Propylenglycolmonostearat, Polyethylenglycol, Polysorbate und Polyvinylalkohol oder eine Mischung dieser ist.

33. Verfahren nach Anspruch 26, wobei dieses ferner das Überziehen der Mikrokapsel mit einem Zellulosepolymerüberzug umfasst.

34. Verfahren nach einem der Ansprüche 26 bis 33, wobei der Wirkstoff ein pharmazeutischer Wirkstoff ist.

35. Verfahren nach Anspruch 34, wobei der Wirkstoff ausgewählt wird aus Nifedipin, Gemfibrizol, Terfenadin, Captopril, Cyclosporin, Calcitonin, Heparinen, Heparinoiden, Amlodipin und Nimodipin.

36. Verfahren nach Anspruch 35, wobei der Wirkstoff Cyclosporin ist.

37. Verfahren nach Anspruch 35, wobei der Wirkstoff Nimodipin ist.

38. Verfahren nach Anspruch 34, wobei der Wirkstoff ein therapeutischer Wirkstoff ist, ausgewählt aus Peptiden, Proteinen, Vakzinen und Oligonukleotiden.

39. Verfahren nach einem der Ansprüche 26 bis 38, ferner umfassend das formulieren der Mikrokapsel in fester Darreichungsform, die eine Mehrzahl derartiger nahtloser Mikrokapseln umfasst.

## Revendications

1. Formulation à libération contrôlée sous forme de dosage unitaire solide, ladite formulation comprenant une multiplicité de microcapsules sans soudure, chaque microcapsule comprenant :
une enveloppe possédant une composition polymérique constituée d'une gélatine souple ou dure ; et
un noyau qui est en contact avec l'enveloppe et est une solution constituée d'un ou de plusieurs ingrédients dissouts dans un glycéride choisi parmi des mono-, di- et/ou triglycérides à chaine moyenne, qui comprend un tensioactif en tant qu'agent auxiliaire
et chaque microcapsule possédant un taux de libération prédéterminé de chaque dit ingrédient actif dans le tractus gastro-intestinal après l'administration, les microcapsules possédant collectivement un ou plusieurs taux de libération de chaque dit ingrédient actif dépendant d'une perméabilité prédéterminée des microcapsules respectives.

2. Formulation selon la revendication 1, dans laquelle les enveloppes des microcapsules incluent également un polymère à libération prolongée.

3. Formulation selon la revendication 1 ou la revendication 2, dans laquelle une partie ou toutes les microcapsules de la formulation possèdent un enrobage entérique.

4. Formulation selon l'une quelconque des revendications précédentes, dans laquelle les microcapsules possèdent un diamètre moyen compris dans la gamme de 0,5 à 5 mm.

5. Formulation selon l'une quelconque des revendications précédentes, dans laquelle le tensioactif est le laurylsulfate de sodium, le monostéarate de diéthylène glycol, le monostéarate de propylène glycol, le polyéthylène glycol, les polysorbates et l'alcool polyvinylique ou un mélange de ceux-ci.

6. Formulation selon la revendication 1, dans laquelle les microcapsules comprennent en outre un enrobage à base de polymère de cellulose.

7. Formulation selon l'une quelconque des revendications précédentes, dans laquelle l'ingrédient actif est un ingrédient pharmaceutique.

8. Formulation selon la revendication 7, dans laquelle l'ingrédient actif est choisi parmi la nifédipine, le gemfibrizol, la terfénadine, le captopril, la cyclosporine, la calcitonine, les héparines, les héparinoïdes, l'amlopidine et la nimodipine.

9. Formulation selon la revendication 8, dans laquelle l'ingrédient actif est la cyclosporine.

10. Formulation selon la revendication 8, dans laquelle l'ingrédient actif est la nimodipine.

11. Formulation selon la revendication 7, dans laquelle l'ingrédient actif est un agent thérapeutique choisi parmi les peptides, les protéines, les vaccins et les oligonucléotides.

12. Formulation selon l'une quelconque des revendications 1 à 7 qui est une formulation à ingrédients actifs multiples.

13. Formulation selon l'une quelconque des revendications 1 à 7, dans laquelle les microcapsules contiennent deux ou plus ingrédients actifs possédant des solubilités différentes dans l'environnement aqueux du tractus gastro-intestinal, mais avec des solubilités comparables dans le milieu liquide de la microcapsule.

14. Formulation selon l'une quelconque des revendications 1 à 7, dans laquelle les microcapsules contiennent deux ou plus ingrédients actifs possédant des demi-vies différentes après absorption par le tractus gastro-intestinal.

15. Microcapsule sans soudure comprenant :
une enveloppe possédant une composition polymérique constituée d'une gélatine souple ou dure ; et
un noyau qui est en contact avec l'enveloppe et est une solution constituée d'un ou de plusieurs ingrédients dissouts dans un glycéride choisi parmi des mono-, di- et/ou triglycérides à chaine moyenne, qui comprend un tensioactif en tant qu'agent auxiliaire
et chaque microcapsule possédant un taux de libération prédéterminé de chaque dit ingrédient actif dans le tractus gastro-intestinal après l'administration, les microcapsules possédant collectivement un ou plusieurs taux de libération de chaque dit ingrédient actif dépendant d'une perméabilité prédéterminée des microcapsules respectives.

16. Microcapsule selon la revendication 15, dans laquelle l'enveloppe inclut également un polymère à libération prolongée.

17. Microcapsule selon la revendication 15 ou la revendication 16 qui possède un enrobage entérique.

18. Microcapsule selon l'une quelconque des revendications 15 à 17 qui possède un diamètre moyen compris dans la gamme de 0,5 à 5 mm.

19. Microcapsule selon l'une quelconque des revendications 15 à 18, dans laquelle le tensioactif est le laurylsulfate de sodium, le monostéarate de diéthylène glycol, le monostéarate de propylène glycol, le polyéthylène glycol, les polysorbates et l'alcool polyvinylique ou un mélange de ceux-ci.

20. Microcapsule selon la revendication 15 qui comprend en outre un enrobage à base de polymère de cellulose.

21. Microcapsule selon l'une quelconque des revendications 15 à 20, dans laquelle l'ingrédient actif est un ingrédient pharmaceutique.

22. Microcapsule selon la revendication 21, dans laquelle l'ingrédient actif est choisi parmi la nifédipine, le gemfibrizol, la terfénadine, le captopril, la cyclosporine, la calcitonine, les héparines, les héparinoïdes, l'amlopidine et la nimodipine.

23. Microcapsule selon la revendication 22, dans laquelle l'ingrédient actif est la cyclosporine.

24. Microcapsule selon la revendication 22, dans laquelle l'ingrédient actif est la nimodipine.

25. Microcapsule selon la revendication 21, dans laquelle l'ingrédient actif est un agent thérapeutique choisi parmi les peptides, les protéines, les vaccins et les oligonucléotides.

26. Procédé de formation d'une microcapsule sans soudure selon la revendication 15, consistant à :
combiner afin de former des gouttelettes (i) une solution principale hydrophobe d'un ou de plusieurs ingrédients actifs dissouts dans un glycéride choisi parmi des mono-, di- et/ou triglycérides qui comprend un tensioactif en tant qu'agent auxiliaire et (ii) une solution d'enveloppe à base de gélatine ; et
passer les gouttelettes à travers une huile ou un autre système de refroidissement.

27. Procédé selon la revendication 26, consistant à éjecter à travers un orifice une solution principale hydrophobe d'un ou de plusieurs ingrédients actifs dissouts dans une phase liquide pharmaceutiquement acceptable qui comprend un tensioactif en tant qu'agent auxiliaire, et à éjecter à travers un orifice externe une enveloppe à base de gélatine afin de former une sphère encapsulée, le sphère étant éjectée dans une solution de refroidissement ou de durcissement et la solution de l'enveloppe externe étant gélifiée ou solidifiée.

28. Procédé selon la revendication 26, consistant à éjecter à travers un embout possédant un unique orifice une suspension à base d'un mélange enveloppe/noyau, le noyau étant une solution principale hydrophobe d'un ou de plusieurs ingrédients actifs dissouts dans une phase liquide pharmaceutiquement acceptable qui comprend un tensioactif en tant qu'agent auxiliaire, l'enveloppe étant une solution d'enveloppe à base de gélatine, afin de former une capsule ou une perle de suspension à base d'un mélange enveloppe/noyau qui est ensuite éjectée dans une solution de refroidissement ou de durcissement et la solution de l'enveloppe est gélifiée ou solidifiée.

29. Procédé selon les revendications 26 à 28, dans laquelle la solution d'enveloppe inclut également un polymère à libération prolongée.

30. Procédé selon l'une quelconque des revendications 26 à 29, qui comprend en outre l'enrobage des microcapsules à l'aide d'un enrobage entérique.

31. Procédé selon l'une quelconque des revendications 26 à 28, dans lequel la microcapsule possède un diamètre compris dans la gamme de 0,5 à 5 mm.

32. Procédé selon l'une quelconque des revendications 26 à 31, dans lequel le tensioactif est le laurylsulfate de sodium, le monostéarate de diéthylène glycol, le monostéarate de propylène glycol, le polyéthylène glycol, les polysorbates et l'alcool polyvinylique ou un mélange de ceux-ci.

33. Procédé selon la revendication 26, qui comprend en outre l'enrobage de la microcapsule à l'aide d'un enrobage à base d'un polymère de cellulose.

34. Procédé selon l'une quelconque des revendications 26 à 33, dans lequel l'ingrédient actif est un ingrédient actif pharmaceutique.

35. Procédé selon la revendication 34, dans lequel l'ingrédient actif est choisi parmi la nifédipine, le gemfibrizol, la terfénadine, le captopril, la cyclosporine, la calcitonine, les héparines, les héparinoïdes, l'amlopidine et la nimodipine.

36. Procédé selon la revendication 35, dans lequel l'ingrédient actif est la cyclosporine.

37. Procédé selon la revendication 35, dans lequel l'ingrédient actif est la nimodipine.

38. Procédé selon la revendication 34, dans lequel l'ingrédient actif est un agent thérapeutique choisi parmi les peptides, les protéines, les vaccins et les oligonucléotides.

39. Procédé selon l'une quelconque des revendications 26 à 38 qui comprend en outre la formulation de la microcapsule sous forme de dosage unitaire solide comprenant une multiplicité de telles microcapsules sans soudure.
